# EUROPÄISCHE PATENTSCHRIFT

(11) **EP 0 861 836 B1**
(45) Veröffentlichungstag und Bekanntmachung des Hinweises auf die Patenterteilung: **29.12.2004**
(21) Anmeldenummer: 98102952.3
(22) Anmeldetag: 20.02.1998
(51) Int. Cl.: C07D 313/08, C07C 311/07, A61K 31/335

(54) **Sulfonamid-substituierte anellierte 7-Ring-Verbindungen mit Kaliumkanal blockierender Wirkung**
Sulfonamido substituted condensed, seven-membered ring compounds with potassium channel blocking activity
Dérivés sulfonamide de composés contenant un cycle à sept cha nonscondensé ayant une activité de bloquage des canaux de potassium

(30) Priorität: 26.02.1997 DE 19707656
(43) Veröffentlichungstag der Anmeldung: 02.09.1998
(73) Patentinhaber: Aventis Pharma Deutschland GmbH, 65929 Frankfurt am Main (DE)
(72) Erfinder: Brendel, Joachim, Dr., 61118 Bad Vilbel (DE); Lang, Hans Jochen, Dr., 65719 Hofheim (DE); Gerlach, Uwe, Dr., 65795 Hattersheim (DE)

(56) Entgegenhaltungen:
- WO-A-94/13292
- WO-A-95/14470
- US-A- 5 258 510
- LOHRMANN E ET AL: "A NEW CLASS OF INHIBITORS OF CAMP-MEDIATED CL- SECRETION IN RABBIT COLON, ACTING BY THE REDUCTION OF CAMP-ACTIVATED K+ CONDUCTANCE" PFLUEGERS ARCHIV, Bd. 429, 1995, Seiten 517-530, XP002038768
- COLATSKY T J ET AL: "POTASSIUM CHANNEL BLOCKERS AS ANTIARRHYTHMIC DRUGS" DRUG DEVELOPMENT RESEARCH, Bd. 33, Nr. 3, 1.November 1994, Seiten 235-249, XP000567177

## Beschreibung

Sulfonamid-substituierte anellierte 7-Ring-Verbindungen, ihre Verwendung als Medikament sowie sie enthaltende pharmazeutische Zubereitungen

Die vorliegende Erfindung betrifft Verbindungen der Formel I, 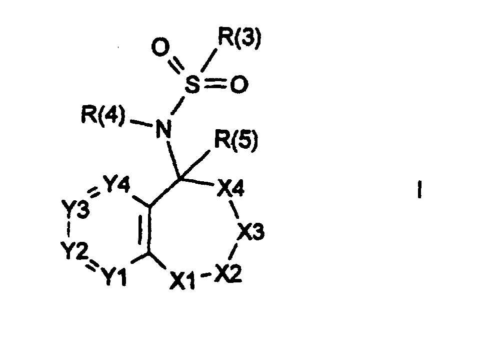 worin X1, X2, X3, X4, Y1, Y2, Y3, Y4, R(3), R(4) und R(5) die im folgenden angegebenen Bedeutungen haben, ihre Herstellung und ihre Verwendung, insbesondere in Arzneimitteln. Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acylaminochroman-Derivate bzw. deren Homologe und Analoga intensiv bearbeitet. Prominentester Vertreter dieser Klasse ist das Cromakalim der Formel A, ein Beispiel für ein Homologes ist die Verbindung der Formel B (J. Chem. Soc., Perkin Trans. 1, 1991, 2763).

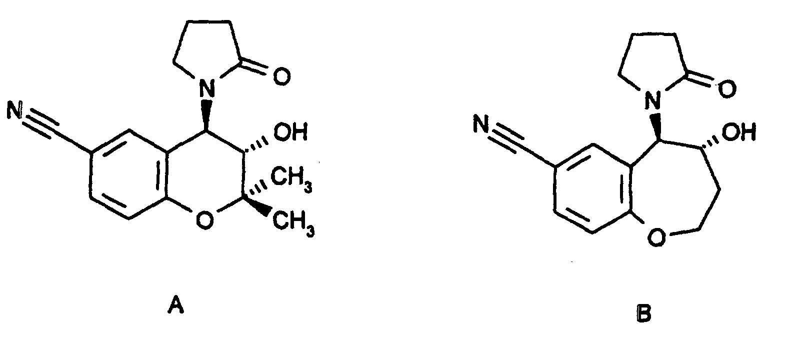

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-lonen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Von diesen Acylamino-Derivaten unterscheiden sich die erfindungsgemäßen Verbindungen der Formel I strukturell insbesondere durch den Ersatz der Acylaminogruppe durch eine Sulfonylaminofunktion. Während das Cromakalim (Formel A) sowie das Homologe der Formel B und analoge Acylaminoverbindungen als Öffner ATP-sensitiver K⁺-Kanäle wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺(cAMP) -Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP) -Kanals bzw. des I_{Ks}-Kanals entwickeln die erfindungsgemäßen Verbindungen im lebenden Organismus pharmakologische Wirkungen von hoher therapeutischer Verwertbarkeit.

Neben den oben genannten Cromakalim- bzw. Acylaminochroman-Derivaten werden in der Literatur auch Verbindungen mit 4-Sulfonylaminochroman-Struktur beschrieben, die sich aber entweder in der Struktur oder in der biologischen Wirkung deutlich von den erfindungsgemäßen Verbindungen der Formel I unterscheiden. So werden in der EP-A-315 009 Chromanderivate mit 4-Phenylsulfonylaminostruktur beschrieben, die sich durch antithrombotische und antiallergische Eigenschaften auszeichnen. In der EP-A-389 861 und der EP-A-370 901 werden 3-Hydroxychroman-Derivate mit einer 4-Sulfonylaminogruppe beschrieben, die als K⁺(ATP)-Kanalaktivatoren beschrieben werden bzw. über ZNS-Wirkungen verfügen. Weitere 4-Sulfonylaminochroman-Derivate werden in Bioorg. Med. Chem. Lett. 4 (1994), 769 - 773: "N-sulfonamides of benzopyran-related potassium channel openers: conversion of glyburyde insensitive smooth muscle relaxants to potent smooth muscle contractors" beschrieben.

Die vorliegende Erfindung betrifft Verbindungen der Formel I, 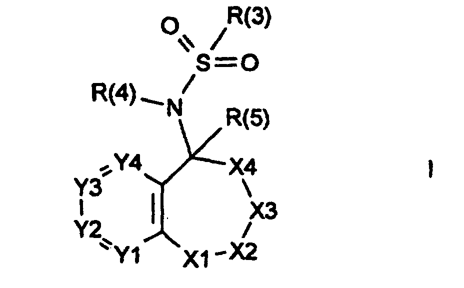
- X1: -O- oder -CR(1)R(2)-;
R(1) und R(2)
unabhängig voneinander Wasserstoff, CF₃, Alkyl mit 1, 2, 3, 4, 5 oder 6 C-Atomen;
- X2: -CR(1)R(2)-,
wobei die Reste R(1) und R(2)
wie bei X1 angegeben definiert sind, aber unabhängig von den Bedeutungen der Reste in X1 sind;
- X3: -CR(1)R(2)-,
wobei die Reste R(1) und R(2)
wie bei X1 angegeben definiert sind, aber unabhängig von den Bedeutungen der Reste in X1 sind;
- X4: -CR(1)R(2)- oder -CH(OR(30))-;
wobei die Reste R(1) und R(2)
wie bei X1 angegeben definiert sind, aber unabhängig von den Bedeutungen der Reste in X1 sind;
R(30) Wasserstoff, Alkyl mit 1, 2 oder 3 C-Atomen oder Acyl mit 1, 2, 3 oder 4 C-Atomen;
- Y1, Y2, Y3 und Y4: unabhängig voneinander -CR(12)-;
die Reste R(12)
unabhängig voneinander Wasserstoff, F, Cl, Br, I, Alkyl mit 1, 2, 3, 4
oder 5 C-Atomen, CN, CF₃, NO₂, -Z-CₘH₂ₘ-R(13) oder Phenyl, das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Z O-, -CO-, -CO-O-, -O-CO-, -S-, -SO-, -SO₂-, -SO₂NR(14)-, -NR(14)- oder -CONR(14)-;
R(14) Wasserstoff oder Alkyl mit 1, 2 oder 3 C-Atomen;
m Null, 1, 2 oder 3;
R(13) Wasserstoff, CF₃, -NR(15)R(16), -CONR(15)R(16), -OR(30a), Phenyl, Thienyl oder einen N-haltigen Heterocyclus mit 1, 2, 3, 4, 5, 6, 7, 8 oder 9 C-Atomen,
wobei Phenyl, Thienyl oder der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2 oder 3 C-Atomen; oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(30a)
Wasserstoff, Alkyl mit 1, 2 oder 3 C-Atomen oder Acyl mit 1, 2, 3 oder 4 C-Atomen;
- R(3): R(17)-CₓH₂ₓ-,
R(17) Methyl, Cycloalkyl mit 3, 4, 5 oder 6 C-Atomen oder CF₃;
x Null, 1, 2, 3, 4, 5, 6, 7, 8, 9 oder 10;
- R(4): -CᵣH₂ᵣ-R(20),
wobei eine CH₂-Gruppe der Gruppe CᵣH₂ᵣ ersetzt sein kann durch -O-, -CO-O, -O-CO-, -NR(21)- oder -CONR(21)-;
R(21) Wasserstoff oder Alkyl mit 1, 2 oder 3 C-Atomen;
r Null, 1, 2, 3, 4, 5, 6, 7, 8, 9 oder 10;
R(20) Methyl, CF₃ oder Cycloalkyl mit 3, 4, 5 oder 6 C-Atomen;
- (R5): Wasserstoff;

in allen ihren stereoisomeren Formen und Gemische davon in beliebigen Verhältnissen, sowie ihre physiologisch verträglichen Salze.

Besonders speziell bevorzugt sind Verbindungen der Formel I, worin bedeuten:
- X1: -O- oder -CH₂;
- X2: -CR(1)R(2)-,
R(1) und R(2)
unabhängig voneinander Wasserstoff oder Alkyl mit 1, 2 oder 3 C-Atomen;
- X3: -CH₂- oder -C(CH₃)₂-;
- X4: -CH₂- oder -CHOH-;
- Y1, Y2, Y3 und Y4: unabhängig voneinander -CR(12)-;
die Reste R(12)
unabhängig voneinander Wasserstoff, F, Cl, Br, Alkyl mit 1, 2 oder 3 C-Atomen, CN, CF₃, NO₂, -Z-CₘH₂ₘ-R(13);
Z -O-, -CO-, -CO-O-, -O-CO-, -S-, -SO-, -SO₂-, -SO₂NR(14)-, -NR(14)- oder -CONR(14)-; R(14) Wasserstoff oder Alkyl mit 1, 2 oder 3 C-Atomen;
m Null, 1, 2 oder 3;
R(13) Wasserstoff, CF₃, -NR(15)R(16), Phenyl, Piperidyl, 1-Pyrrolidinyl, 4-Morpholinyl, 4-Methylpiperazin-1-yl, Pyridyl, Thienyl oder Imidazolyl;
R(15) und R(16)
unabhängig voneinander Wasserstoff oder Alkyl mit 1, 2 oder 3 C-Atomen;
- R(3): R(17)-CₓH₂ₓ-,
R(17) Methyl;
x Null, 1, 2 oder 3;
- R(4): -CᵣH₂ᵣ-R(20),
wobei eine CH₂-Gruppe der Gruppe CᵣH₂ᵣ ersetzt sein kann durch -O-, -CO-O-, -O-CO-, -NR(21)- oder -CONR(21)-; R(21) Wasserstoff oder Alkyl mit 1, 2 oder 3 C-Atomen;
r Null, 1, 2, 3, 4, 5 oder 6;
R(20) Methyl, CF₃ oder Pyridyl;
- R(5): Wasserstoff;
in allen ihren stereoisomeren Formen und Gemische davon in beliebigen Verhältnissen, sowie ihre physiologisch verträglichen Salze.

Ganz besonders speziell bevorzugt sind Verbindungen der Formel I, in welcher bedeuten:
- X1: -O-;
- X2: -CR(1)R(2)-,
R(1) und R(2)
unabhängig voneinander Wasserstoff oder Alkyl mit 1, 2 oder 3 C-Atomen;
- X3: -CH₂ oder -C(CH₃)₂-;
- X4: -CH₂-;
- Y1: CH;
- Y2: CH;
- Y4: CH;
- Y3: -CR(12)-;
R(12)
F, Cl, Br, Alkyl mit 1, 2 oder 3 C-Atomen, CN, CF₃, NO₂, -Z-CₘH₂ₘ-R(13);
Z -O-, -CO-, -CO-O-, -O-CO-, -S-, -SO-, -SO₂-, -SO₂NR(14)- oder -CONR(14)-;
R(14) Wasserstoff oder Alkyl mit 1, 2 oder 3 C-Atomen;
m 1, 2 oder 3;
R(13) Wasserstoff, CF₃, Pyridyl oder Phenyl;
- R(3): R(17)-CₓH₂ₓ-,
R(17) Methyl;
x Null, 1 oder 2;
- R(4): -CᵣH₂ᵣ-R(20),
wobei eine CH₂-Gruppe der Gruppe CᵣH₂ᵣ ersetzt sein kann durch -O-, -CO-O-, -O-CO- oder -CONR(21)-;
R(21) Wasserstoff oder Alkyl mit 1, 2 oder 3 C-Atomen;
r Null, 1, 2, 3, 4, 5 oder 6;
R(20) Methyl oder CF₃;
- R(5): Wasserstoff;
in allen ihren stereoisomeren Formen und Gemische davon in beliebigen Verhältnissen, sowie ihre physiologisch verträglichen Salze.

Alkylreste und Alkylenreste können geradkettig oder verzweigt sein. Dies gilt auch für die Alkylenreste der Formeln CₙH₂ₙ, CₘH₂ₘ, CₓH₂ₓ und CᵣH₂ᵣ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste. Beispiele für Acylreste sind Formyl, Acetyl, Propionyl, n-Butyryl oder Isobutyryl.

Als N-haltige Heterocyclen mit 1, 2, 3, 4, 5, 6, 7, 8 oder 9 C-Atomen gelten insbesondere die aromatischen Systeme 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, - 3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Thienyl steht sowohl für 2- als auch 3-Thienyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position. Entsprechendes gilt sinngemäß analog auch für die N-haltigen Heterocyten oder den Thiophenrest.

Bei Disubstitution eines Restes können die Substituenten gleich oder verschieden sein.

Enthalten die Verbindungen der Formel I eine oder mehrere saure oder basische Gruppen bzw. einen oder mehrere basische Heterocyclen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Bei Vorliegen einer cis/trans-isomerie ist sowohl die cis-Form als auch die trans-Form und Gemische dieser Formen von der Erfindung umfaßt. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar. So erhält man beispielsweise eine Verbindung der Formel I, indem man
a) eine Verbindung der Formel II a oder der Formel II b, 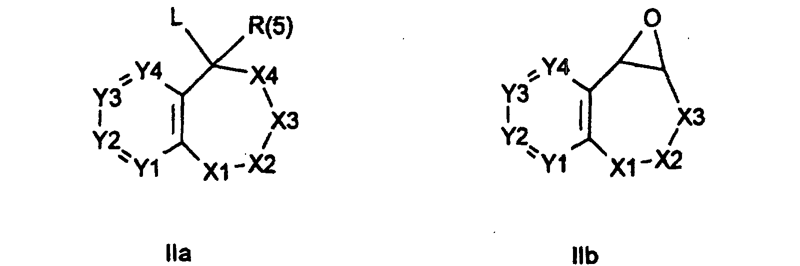 worin X1, X2, X3, X4, Y1, Y2, Y3, Y4 und R(5) die oben angegebenen Bedeutungen besitzen und L eine nucleofuge Fluchtgruppe, insbesondere eine übliche nucleofuge Fluchtgruppe wie z. B. F, Cl, Br, I, Methansulfonyloxy oder p-Toluolsulfonyloxy, bedeutet, in an sich bekannter Weise umsetzt mit einem Sulfonamid oder dessen Salz der Formel III, 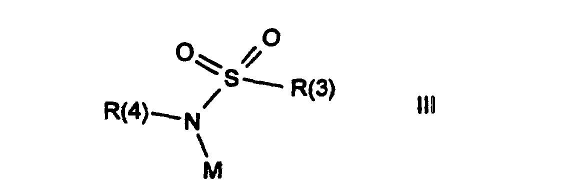 worin R(3) und R(4) die oben angegebenen Bedeutungen besitzen und M für Wasserstoff oder bevorzugt für ein Metallatom, besonders bevorzugt für Lithium, Natrium oder Kalium, steht, oder im Falle einer Umsetzung mit einer Verbindung der Formel II b auch für einen Trialkylsilylrest, z. B. einen Trimethylsilylrest, steht;
   oder daß man
b) eine Verbindung der Formel IV, 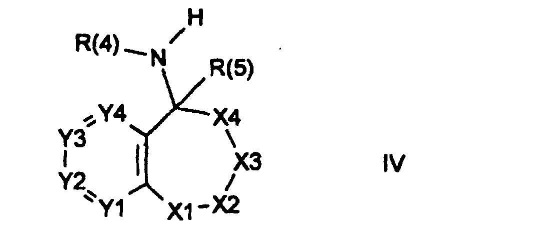 worin X1, X2, X3, X4, Y1, Y2, Y3, Y4, R(4) und R(5) die oben angegebenen Bedeutungen besitzen, mit einem Sulfonsäure-Derivat der Formel V 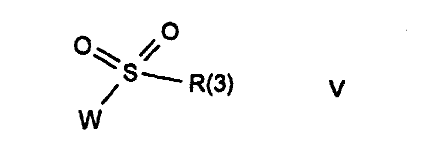 umsetzt, worin R(3) die oben angegebenen Bedeutungen besitzt und Weine nucleofuge Fluchtgruppe, wie z. B. Fluor, Brom, 1-Imidazolyl, insbesondere aber Chlor, bedeutet;
   oder daß man
c) eine Verbindung der Formel VI, 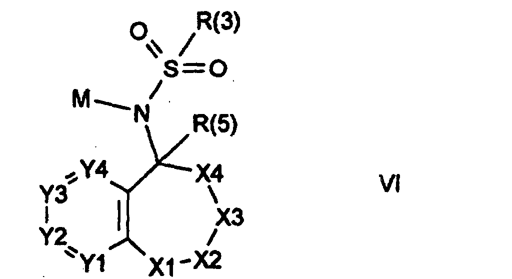 worin X1, X2, X3, X4, Y1, Y2, Y3, Y4, R(3), R(5) und M die oben angegebenen Bedeutungen besitzen, in an sich bekannter Weise im Sinne einer Alkylierungsreaktion mit einem Alkylierungsmittel der Formel VII

   R(4)-L VII

   umsetzt, worin R(4) die oben angegebenen Bedeutungen mit Ausnahme von Wasserstoff besitzt und L die oben angegebenen Bedeutungen besitzt;
   oder daß man
d) in einer Verbindung der Formel I, 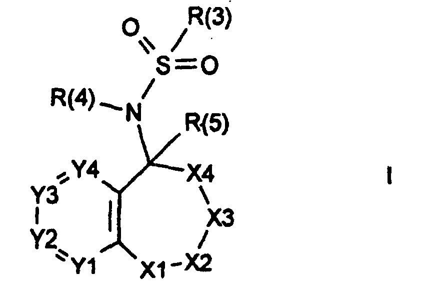 worin X1, X2, X3, X4, Y1, Y2, Y3, Y4, R(3), R(4) und R(5) die oben angegebenen Bedeutungen besitzen, in mindestens einer der Positionen Y1 bis Y4, sofern in dieser Position eine CH-Gruppe vorliegt, eine elektrophile Substitutionsreaktion durchführt, oder in mindestens einer der Positionen X1 bis X4 eine chemische Umwandlung, z.B. eine Alkylierung oder Acylierung, durchführt.

Die Verfahrensweise a) entspricht der nucleophilen Substitution einer Abgangsgruppe in einem reaktiven Bicyclus der Formel II a oder II b durch ein Sulfonamid bzw. eines seiner Salze der Formel III. Wegen der höheren Nucleophilie und höheren Reaktivität eines in der Salzform vorliegenden Sulfonamids ist bei Verwendung eines freien Sulfonamids (Formel III, M = H) bevorzugt, aus diesem zunächst durch Einwirkung einer Base ein Sulfonamidsalz (Formel III, M = Metallkation) zu erzeugen. Setzt man ein freies Sulfonamid (Formel III, M = H) ein, so kann die Deprotonierung des Sulfonamids zum Salz in situ erfolgen. Bevorzugt werden solche Basen verwandt, die selbst nicht oder nur wenig alkyliert werden, wie z. B. Natriumcarbonat, Kaliumcarbonat, sterisch stark gehinderte Amine, z. B. Dicyclohexylamin, N,N-Dicyclohexyl-ethylamin, oder andere starke Stickstoffbasen mit geringer Nucleophilie, beispielsweise DBU (Diazabicycloundecen), N,N',N'''-Triisopropylguanidin etc. Es können allerdings auch andere üblich verwendete Basen für die Reaktion eingesetzt werden, wie Kalium-tert-butylat, Natriummethylat, Alkalihydrogencarbonate, Alkalihydroxide, wie beispielweise LiOH, NaOH oder KOH, oder Erdalkalihydroxide, wie beispielsweise Ca(OH)₂. Für den Fall einer Umsetzung eines Epoxids der Formel II b mit einem Trialkyl- oder Trimethylsilylsulfonamid der Formel III ist es vorteilhaft, die Reaktion in Gegenwart eines Fluorids, z. B. Tetrabutylammoniumfluorid, durchzuführen.

Vorzugsweise arbeitet man in einem Lösungsmittel, besonders bevorzugt in polaren organischen Lösungsmitteln wie z. B. Dimethylformamid (DMF), Dimethylacetamid (DMA), Dimethylsulfoxid (DMSO), Tetramethylharnstoff (TMU), Hexamethylphosphorsäuretriamid (HMPT), Tetrahydrofuran (THF), Dimethoxyethan (DME) oder anderen Ethem, oder beispielsweise auch in einem Kohlenwasserstoff wie Toluol oder in einem halogenierten Kohlenwasserstoff wie Chloroform oder Methylenchlorid usw. Es kann aber auch in polaren protischen Lösungsmitteln gearbeitet werden, wie z. B. in Wasser, Methanol, Ethanol, Isopropanol, Ethylenglykol oder dessen Oligomeren und deren entsprechenden Halbethern oder auch deren Ethem. Die Reaktion kann auch in Gemischen dieser Lösungsmittel durchgeführt werden. Ebenso kann die Reaktion aber auch ganz ohne Lösungsmittel durchgeführt werden. Die Reaktion wird bevorzugt in einem Temperaturbereich von -10 bis +140 °C, besonders bevorzugt im Bereich von 20 bis 100 °C durchgeführt. In günstiger Weise kann Verfahrensweise a) auch unter den Bedingungen einer Phasentransferkatalyse durchgeführt werden.

Die Verbindungen der Formel II a gewinnt man nach literaturbekannten Methoden, beispielsweise aus den entsprechenden Alkoholen (Formel II a, L = OH, R(5) = H) durch Einwirkung eines Halogenwasserstoffs der Formel HL (L = Cl, Br, I) oder durch Einwirkung eines anorganischen Säurehalogenids (z. B. POCl₃, PCl₃, PCl₅, SOCl₂, SOBr₂), oder z. B. durch radikalische Halogenierung der entsprechenden Verbindungen der Formel II a mit L = H und R(5) = H mit elementarem Chlor oder Brom oder mit radikalisch aktivierbaren Halogenierungsmitteln wie z. B. N-Bromsuccinimid (NBS) oder Sulfurylchlorid (SO₂Cl₂). Im Falle der radikalischen Halogenierung wird im allgemeinen in Gegenwart eines Radikalkettenstarters wie energiereichem Licht des sichtbaren oder ultravioletten Wellenbereichs oder unter Verwendung eines chemischen Radikalstarters wie z. B. Azodiisobutyronitril gearbeitet.

Die Verbindungen der Formel II a, in der X4 für -NR(11)- steht und R(5) gemeinsam mit R(11) eine Bindung bedeutet, erhält man beispielsweise aus den entsprechenden Lactamen (Formel II a, L und R(5) bedeuten gemeinsam ein Carbonylsauerstoffatom, X4 = NH) durch Einwirkung eines anorganischen Säurehalogenids (z. B. POCl₃, PCl₃, PCl₅, SOCl₂, SOBr₂).

Die Verbindungen der Formel II b erhält man nach literaturbekannten Methoden aus den entsprechenden Olefinen der Formel II c, 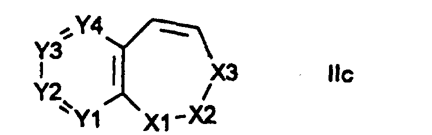 worin X1, X2, X3, Y1, Y2, Y3 und Y4 die oben angegebenen Bedeutungen besitzen, z. B. durch Einwirkung eines geeigneten anorganischen oder organischen Peroxids, wie beispielsweise H₂O₂ oder m-Chlorperbenzoesäure, oder durch basenkatalysierte Cyclisierung des entsprechenden Bromhydrins, das aus II c z. B. durch Umsetzung mit N-Bromsuccinimid und Wasser erhalten werden kann.

Die Epoxide der Formel II b können aus den Olefinen der Formel II c auch in optisch reiner Form erhalten werden durch Oxidation in Gegenwart des chiralen Jacobsen-Katalysators, wie z. B. in Tetrahedron Lett. 32, 1991, 5055 beschrieben.

Die Verfahrensweise b) entspricht der an sich bekannten und häufig angewandten Reaktion einer reaktiven Sulfonylverbindung der Formel V, insbesondere einer Chlorsulfonylverbindung (W = CI), mit einem Amino-Derivat der allgemeinen Formel IV zum entsprechenden Sulfonamid-Derivat der allgemeinen Formel I. Die Reaktion kann auch ohne Lösungsmittel durchgeführt werden, jedoch werden derartige Reaktionen in den meisten Fällen unter Verwendung eines Lösungsmittels durchgeführt. Die Reaktion wird bevorzugt unter Verwendung eines polaren Lösungsmittels durchgeführt, besonders bevorzugt in Gegenwart einer Base, die selbst vorteilhaft als Lösungsmittel verwendet werden kann, z. B. unter Verwendung von Triethylamin oder insbesondere von Pyridin oder dessen Homologen. Geeignete Lösungsmittel sind beispielsweise Wasser, aliphatische Alkohole, z. B. Methanol, Ethanol, Isopropanol, sec-Butanol, Ethylenglykol, Monoalkyl- und Dialkylether des monomeren und oligomeren Ethylenglykols, Tetrahydrofuran, Dioxan, dialkylierte Amide wie DMF, DMA, sowie TMU und HMPT. Man arbeitet dabei im allgemeinen bei einer Temperatur von 0 bis 160 °C, vorzugsweise von 20 bis 100 °C.

Die Amine der Formel IV erhält man in literaturbekannter Weise bevorzugt aus den entsprechenden Carbonylverbindungen der Formel VIII, 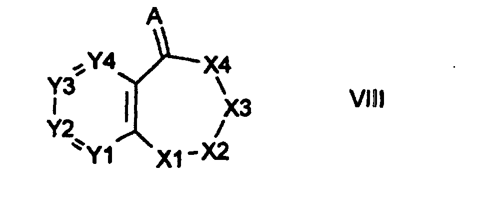 worin X1, X2, X3, X4, Y1, Y2, Y3 und Y4 die oben angegebenen Bedeutungen besitzen und A Sauerstoff bedeutet, entweder mit Ammoniak oder einem Amin der Formel IX,

R(4)-NH₂ IX

in der R(4) die angegebenen Bedeutungen hat, unter reduktiven Bedingungen oder reduktiven katalytischen Bedingungen, vorzugsweise bei höherer Temperatur und im Autoklaven. Dabei werden primär durch Kondensationsreaktion der Ketone der Formel VIII (A = Sauerstoff) und der Amine der Formel IX in situ Schiff-Basen der Formel VIII, in der A für R(4)-N= steht, gebildet, die unmittelbar, d. h. ohne vorherige Isolierung, reduktiv in die Amine der Formel IV überführt werden können. Es können aber auch die bei der Kondensationsreaktion intermediär aus den Verbindungen der Formel VIII und IX entstehenden Schiff-Basen (Formel VIII, A steht für R(4)-N=) nach literaturbekannten Methoden hergestellt und zunächst isoliert werden, um sie dann in einem separaten Schritt mit einem geeigneten Reduktionsmittel, wie z. B. NaBH₄, LiAlH₄, NaBH₃CN oder durch katalytische Hydrierung in Gegenwart von z. B. Raney-Nickel oder einem Edelmetall wie z. B. Palladium, in die Verbindungen der Formel IV umzuwandeln.

Die Verbindungen der Formel IV, in der R(4) für Wasserstoff steht, können vorteilhaft in literaturbekannter Weise auch durch Reduktion von Oximen oder Oximethem (Formel VIII, A steht für =N-OR, R = H oder Alkyl) oder Hydrazonen (Formel VIII, A steht für =N-NR₂, R z. B. = H oder Alkyl) erhalten werden, z. B. unter Verwendung eines komplexen Metallhydrids oder durch katalytische Hydrierung. Die dafür erforderlichen Oxime und Hydrazone werden vorzugsweise in an sich bekannter Weise aus den Ketonen der Formel VIII (A = Sauerstoff) mit Hydrazin oder einem seiner Derivate oder beispielsweise mit Hydroxylamin-Hydrochlorid unter wasserabspaltenden Bedingungen hergestellt.

Die Verfahrensweise c) repräsentiert die an sich bekannte Alkylierungsreaktion eines Sulfonamids bzw. eines seiner Salze (Formel VI) mit einem Alkylierungsmittel (Formel VII). Entsprechend der Reaktionsanalogie mit Verfahrensweise a) gelten für Verfahrensweise c) die bereits ausführlich unter Verfahrensweise a) beschriebenen Reaktionsbedingungen.

Die Herstellung der Sulfonamid-Derivate der Formel VI und deren Vorprodukte wurde bereits bei Verfahrensweise b) beschrieben. Die Herstellung der Alkylantien der Formel VII erfolgt analog zu Vorschriften in der Literatur bzw. wie unter Verfahrensweise a) beschrieben, vorzugsweise aus den entsprechenden Hydroxyverbindungen (Formel VII, L = Hydroxy).

Die Verfahrensweise d) entspricht der weiteren chemische Umwandlung von erfindungsgemäßen Verbindungen der Formel I in andere erfindungsgemäße Verbindungen der Formel I, z. B. durch elektrophile Substitutionsreaktionen in einer oder in mehreren der mit Y1 bis Y4 bezeichneten Positionen, sofern diese CH bedeuten. Bevorzugte Substitutionsreaktionen sind
1. die aromatische Nitrierung zur Einführung einer oder mehrerer Nitrogruppen, die in nachfolgenden Reaktionen teilweise oder alle zu Aminogruppen reduziert werden können. Die Aminogruppen können wiederum in nachfolgenden Reaktionen in andere Gruppen umgewandelt werden, beispielsweise in einer Sandmeyerreaktion, z. B. zur Einführung von Cyanogruppen;
2. die aromatische Halogenierung, insbesondere zur Einführung von Chlor, Brom oder Jod;
3. die Chlorsulfonierung, z. B. durch Einwirkung von Chlorsulfonsäure, zur Einführung einer Chlorsulfonylgruppe, die in nachfolgenden Reaktionen in andere Gruppen umgewandelt werden kann, z. B. in eine Sulfonamidgruppe;
4. die Friedel-Crafts-Acylierungsreaktion zur Einführung eines Acylrestes oder eines Sulfonylrestes durch Einwirkung der entsprechenden Säurechloride in Gegenwart einer Lewis-Säure als Friedel-Crafts-Katalysator, vorzugsweise in Gegenwart von wasserfreiem Aluminiumchlorid.

Als weitere chemische Umwandlungen von erfindungsgemäßen Verbindungen der Formel I sind z. B. Reaktionen in den Positionen X1 bis X4 möglich. So können z. B. erfindungsgemäße Verbindungen der Formel I (X4 = CHOH) durch Alkylierung oder Acylierung mit einer Verbindung der Formel L-R(30), wobei L die oben angegebenen Bedeutungen hat und R(30) die oben angegebenen Bedeutungen mit Ausnahme von Wasserstoff hat, in die entsprechen Alkyl- oder Acylderivate der Formel I (X4 = CHOR(30)) überführt werden. Weiterhin kann z. B. die Hydroxygruppe durch wasserabspaltende Mittel eliminiert werden, so daß eine Verbindung der Formel 1, in der R(5) und R(11) gemeinsam eine Bindung bilden, erhalten wird.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Die für die obigen Verfahren erforderlichen bicyclischen Vorstufen sind entweder literaturbekannt oder lassen sich analog bekannten Verfahren herstellen. Einige Methoden zur Herstellung dieser Vorstufen sind z.B. in folgenden Literaturstellen beschrieben, die insofern vollinhaltlich Bestandteil der vorliegenden Offenbarung sind: J. Chem. Soc., Perkin Trans. 1, 1991, 2763; Eur. J. Med. Chem. 30 (1995), 377; Ind. J. Chem. 9 (1971), 809; J. Heterocyclic Chem. 26 (1989), 1547.

Es wurde bereits gesagt, daß die Verbindungen der Formel I überraschenderweise eine starke und spezifische blockierende (schließende) Wirkung auf einen K⁺-Kanal haben, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine antidiarrhoische Wirkung aus und sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien, vor allem von Herzrhythmusstörungen, die durch Aktionspotential-Verlängerung behoben werden können. Sie können speziell angewandt werden zur Therapie und Prophylaxe von atrialer Fibrillation (Vorhofflimmern) und atrialem Flattern (Vorhofflattem) sowie zur Therapie und Prophylaxe von Reentry-Arrhythmien und zur Verhinderung des plötzlichen Herztodes infolge von Kammerflimmem.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht.
Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III-Antiarrhythmika blockieren überwiegend oder ausschließlich I_{Kr} (z.B. Dofetilid, d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44B - 49B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T.J. Colatsky, C.H. Follmer und C.F. Starmer; "Channel Specificity in Antiarrhythmic Drug Action; Mechanism of potassium channel block and its role in suppressing and aggravating cardiac arrhythmias"; Circulation 82 (1990), 2235 - 2242; A.E. Busch, K. Malloy, W.J. Groh, M.D. Varnum, J.P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys. Res. Commun. 202 (1994), 265 - 270).

Darüber hinaus tragen die Verbindungen I zu einer deutlichen Verbesserung der Herzinsuffizienz bei, insbesondere der Stauungsinsuffizienz (congestive heart failure), vorteilhaft in der Kombination mit kontraktionsfördemden (positiv inotropen) Wirkstoffen, zum Beispiel Phosphordiesterasehemmem.

Trotz der therapeutisch nutzbaren Vorteile, die durch eine Blockade des I_{Ks} erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1:10). In der WO-A-95/14470 wird die Verwendung von Benzodiazepinen als selektive Blocker des I_{Ks} beansprucht.

Weitere I_{Ks}-Blocker sind beschrieben in FEBS Letters 396 (1996), 271 - 275: "Specific blockade of slowly activating I_{sK} channels by chromanols ..." und Pflügers Arch. - Eur. J. Physiol. 429 (1995), 517 - 530: "A new class of inhibitors of cAMPmediated Cl⁻ secretion in rabbit colon, acting by the reduction of cAMP-activated K⁺ conductance". Die Potenz der dort genannten Verbindungen ist jedoch deutlich geringer als die der erfindungsgemäßen Verbindungen der Formel I.

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnem, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Exchange-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H₂-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

Die Verbindungen der Formel I und ihre physiologisch verträglichen Salze hemmen selektiv K⁺(cAMP)-Kanäle bzw. I_{Ks}-Kanäle. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine Beeinflussung von Kaliumkanälen beabsichtigt ist, sowie für diagnostische Zwecke, z. B. in der in vitro-Diagnostik von Zell- oder Gewebsproben. Ferner können sie, wie bereits oben erwähnt, als Zwischenprodukte zur Herstellung weiterer Arzneimittelwirkstoffe eingesetzt werden.

### Experimenteller Teil

### Liste der Abkürzungen

- DMA: N,N-Dimethylacetamid
- DMSO: Dimethylsulfoxid
- EE: Essigsäurethylester
- Fp: Schmelzpunkt [Wenn nicht anders angegeben, sind die Schmelzpunkte der ungereinigten Rohprodukte angegeben; die Schmelzpunkte der jeweiligen Reinsubstanzen können durchaus deutlich höher liegen].
- NBS: N-Bromsuccinimid
- RT: Raumtemperatur
- THF: Tetrahydrofuran

### Beispiel 1: 3-Fluor-5-(N-ethylsulfonylamino)-6,7,8,9-tetrahydro-5H-benzocyclohepten

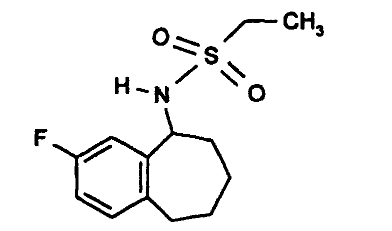

a) 5 g (28 mmol) 8-Fluor-1-benzosuberon und 2,1 g (31 mmol) Hydroxylamin-Hydrochlorid wurden in 20 ml Ethanol und 20 ml Pyridin 5 h unter Rückfluß erhitzt. Nach Abdestillieren der Lösungsmittel am Rotationsverdampfer wurde der Rückstand mit Wasser versetzt, mit verd. Salzsäure auf pH 2 gestellt und bis zur Kristallisation des anfänglich öligen Produktes gerührt. Man erhielt 5,1 g 8-Fluor-1-benzosuberonoxim (Fp 97 - 103 °C), das durch Hydrierung in Methanol bei Normaldruck und RT mit Raney-Nickel als Katalysator zu 3-Fluor-5-amino-6,7,8,9-tetrahydro-5H-benzocyclohepten reduziert wurde. Ausbeute: 4,3 g; Fp des Hydrochlorids 212 - 216 °C.
b) Zu einer Lösung von 2,68 g (15 mmol) 3-Fluor-5-amino-6,7,8,9-tetrahydro-5Hbenzocyclohepten und 1,5 g (60 mmol) Triethylamin in 40 ml THF wurden unter Eiskühlung 2,3 g (18 mmol) Ethansulfonsäurechlorid zugetropft. Man ließ auf Raumtemperatur kommen, rührte über Nacht nach und destillierte das Lösungsmittel im Vakuum ab. Nach Verrühren des Rückstandes mit Wasser wurde das ausgefallene Produkt abgesaugt. Man erhielt 2,9 g 3-Fluor-5-(N-ethylsulfonylamino)-6,7,8,9-tetrahydro-5H-benzocyclohepten. Fp 118 -121 °C.

### Beispiel 2: 3-Fluor-5-(N-ethylsulfonyl-N-methylamino)-6,7,8,9-tetrahydro-5Hbenzocyclohepten

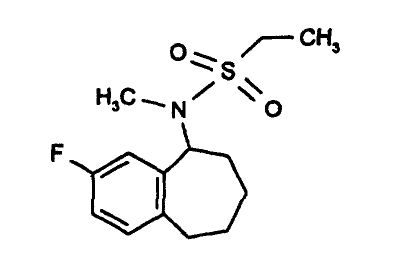

Zu einer Suspension von 0,12 g (4,1 mmol) 80proz. Natriumhydrid in 5 ml DMA wurde unter Argon eine Lösung von 0,81 g (3 mmol) 3-Fluor-5-(Nethylsulfonylamino)-6,7,8,9-tetrahydro-5H-benzocyclohepten (Beispiel 1) in 10 ml DMA zugetropft. Nach 3 h Rühren bei RT tropfte man 0,63 g (4,5 mmol) Methyliodid zu und ließ über Nacht bei RT weiterrühren. Nach Abdestillieren des Lösungsmittels wurde der Rückstand mit Wasser versetzt und mit EE extrahiert. Die organische Phase wurde nach Trocknung über Natriumsulfat im Vakuum eingeengt Man erhielt 0,64 g 3-Fluor-5-(N-ethylsulfonyl-N-methylamino)-6,7,8,9-tetrahydro-5Hbenzocyclohepten.

### Beispiel 3: 3-Fluor-5-(N-ethylsulfonyl-N-butylamino)-6,7,8,9-tetrahydro-5Hbenzocyclohepten

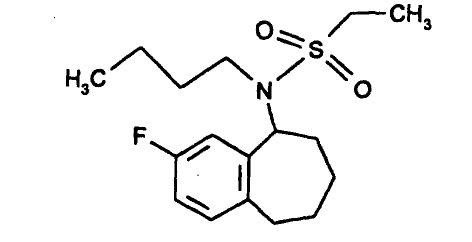

Aus 0,12 g (4,1 mmol) 80proz. Natriumhydrid, 0,81 g (3 mmol) 3-Fluor-5-(Nethylsulfonylamino)-6,7,8,9-tetrahydro-5H-benzocyclohepten und 0,82 g (4,5 mmol) Butyliodid wurden analog Beispiel 2 0,88 g 3-Fluor 5-(N-ethylsulfonyl-N-butylamino)-6,7,8,9-tetrahydro-5H-benzocyclohepten erhalten.

### Beispiel 4: trans-7-Nitro-5-(N-ethylsulfonyl-N-methylamino)-2,3,4,5-tetrahydro-1-benzoxepin-4-ol

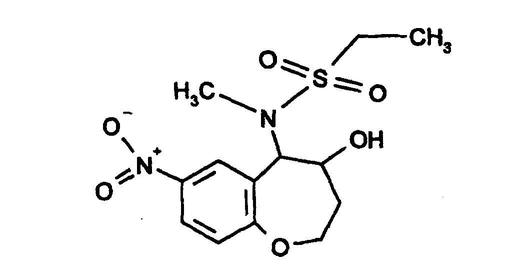

a) 2,3-Dihydro-7-nitro-1-benzoxepin
   Zu einer Suspension von 50 g 3,4-Dihydro-7-nitro-1-benzoxepin-5(2H)-on (J. Chem. Soc., Perkin Trans. 1, 1991, 2763) in 345 ml Methanol wurden bei 0 °C unter starkem Rühren 10,2 g Natriumboranat portionsweise hinzugefügt. Nach 30 min gab man das Reaktionsgemisch auf Eiswasser, saugte den Feststoff ab und erhielt 43 g 7-Nitro-2,3,4,5-tetrahydro-1-benzoxepin-5-ol. Dieses wurde mit 1 g p-Toluolsulfonsäure in 520 ml Toluol 2 h am Wasserabscheider erhitzt. Nach dem Abkühlen wurde die organische Phase mit Natriumbicarbonatlösung und Kochsalzlösung gewaschen. Nach Trocknen über Natriumsulfat und Einengen erhielt man 38,8 g 2,3-Dihydro-7-nitro-1-benzoxepin. Fp 98 -100 °C.
b) 4,5-Epoxy-7-nitro-2,3,4,5-tetrahydro-1-benzoxepin
   Zu einer Lösung von 38,8 g 2,3-Dihydro-7-nitro-1-benzoxepin in 780 ml DMSO und 78 ml Wasser wurden unter Eiskühlung 72,3 g NBS auf einmal zugefügt, wobei die Temperatur bis auf 30 °C anstieg. Nach 90 min Nachrühren bei RT goß man das Reaktionsgemisch in 5 I Eiswasser, rührte 1 h nach, saugte den kristallinen Niederschlag ab und erhielt 60 g des Bromhydrins. Eine Lösung dieses Bromhydrins in 465 ml Methanol wurde bei RT zu einer Lösung von 5,1 g Natrium in 400 ml Methanol getropft. Nach 1 h Rühren wurde der ausgefallene Niederschlag abgesaugt und mit Wasser gewaschen. Nach dem Trocknen erhielt man 37 g 4,5-Epoxy-7-nitro-2,3,4,5-tetrahydro-1-benzoxepin. Fp 123 - 125°C.
c) N-Methyl-N-trimethylsilyl-ethansulfonamid
   Eine Mischung von 33 g N-Methyl-ethansulfonamid, 101 g Hexamethyldisilazan und einer Spatelspitze Ammoniumchlorid wurde unter Argon zunächst 2 h auf 100 °C erhitzt und dann noch 1 h auf 130 °C erhitzt. Durch Destillation der Reaktionsmischung im Vakuum erhielt man beim Siedepunkt 118 - 121 °C /10 Torr 42 g N-Methyl-N-trimethylsilyl-ethansulfonamid.
d) trans-7-Nitro-5-(N-ethylsulfonyl-N-methylamino)-2,3,4,5-tetrahydro-1-benzoxepin-4-ol
   Zu einer Mischung von 6 g 4,5-Epoxy-7-nitro-2,3,4,5-tetrahydro-1-benzoxepin und 9,5 g N-Methyl-N-trimethylsilyl-ethansulfonamid wurden unter Rühren 2 g Tetrabutylammoniumfluorid zugegeben. Dann erhitzte man 3 h auf 65 °C, rührte über Nacht bei RT und goß die Reaktionsmischung auf Ammoniumchloridlösung. Man extrahierte mehrmals mit EE, wusch die organische Phase mit Wasser und trocknete über Natriumsulfat. Nach Einengen und Umkristallisation des festen Rückstandes aus Isopropanol erhielt man 7,1 g trans-7-Nitro-5-(N-ethylsulfonyl-N-methylamino)-2,3,4,5-tetrahydro-1-benzoxepin-4-ol. Fp 133 - 135 °C.

### Beispiel 5: trans-7-Cyano-5-(N-ethylsulfonyl-N-methylamino)-2,3,4,5-tetrahydro-1-benzoxepin-4-ol

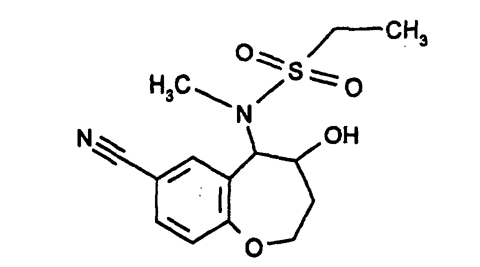

Zu einer Mischung von 4 g 4,5-Epoxy-2,3,4,5-tetrahydro-1-benzoxepin-7-carbonitril (J. Chem. Soc., Perkin Trans. 1, 1991, 2763) und 7,0 g N-Methyl-N-trimethylsilyl-ethansulfonamid wurden unter Rühren 1,5 g Tetrabutylammoniumfluorid zugegeben. Dann erhitzte man 3 h auf 60 °C, rührte über Nacht bei RT und goß die Reaktionsmischung auf Ammoniumchloridlösung. Man extrahierte mehrmals mit EE, wusch die organische Phase mit Wasser und trocknete über Natriumsulfat. Nach Einengen und Umkristallisation des festen Rückstandes aus Cyclohexan/EE (1:5) erhielt man 3,2 g trans-7-Cyano-5-(N-ethylsulfonyl-N-methylamino)-2,3,4,5-tetrahydro-1-benzoxepin-4-ol. Fp 142 -144 °C.

### Beispiel 6: 5-(N-Ethylsulfonyl-N-methylamino)-2,3,4,5-tetrahydro-1-benzoxepin

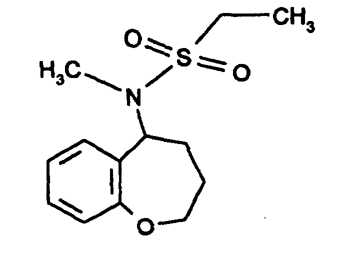

a) Eine Lösung von 10,0 g (62 mmol) 3,4-Dihydro-1-benzoxepin-5(2H)-on (J. Chem. Soc., Perkin Trans. 1, 1991, 2763) und 4,63 g (68 mmol) Hydroxylamin-Hydrochlorid in 45 ml Ethanol und 45 ml Pyridin wurde 5 h unter Rückfluß erhitzt. Nach Abdestillieren der Lösungsmittel am Rotationsverdampfer wurde der Rückstand mit Wasser versetzt, mit verd. Salzsäure auf pH 2 gestellt und 3 h nachgerührt. Nach Absaugen und Trocknen des ausgefallenen Produktes erhielt man 10,2 g 3,4-Dihydro-1-benzoxepin-5(2H)-on-oxim. Fp 96 - 98 °C.
b) Eine Lösung von 2,0 g (11,3 mmol) 3,4-Dihydro-1-benzoxepin-5(2H)-on-oxim in 15 ml 1,2-Dimethoxyethan (DME) wurde bei 0 °C unter Argon zu einer Mischung aus 4,5 g (23,7 mmol) Titantetrachlorid und 1,79 g (47,4 mmol) Natriumboranat in 50 ml DME in 20 min zugetropft. Nach 2 Tagen Rühren bei RT wurden 100 ml Wasser zugetropft und mit konz. Ammoniaklösung alkalisch gestellt. Nach Absaugen des Niederschlages wurde das Filtrat dreimal mit EE extrahiert. Nach Waschen mit Kochsalzlösung, Trocknen über Magnesiumsulfat und Einengen erhielt man 2,1 g 5-Amino-2,3,4,5-tetrahydro-1-benzoxepin.
c) Zu einer Lösung von 1,0 g (6,1 mmol) 5-Amino-2,3,4,5-tetrahydro-1-benzoxepin und 2,5 g (24 mmol) Triethylamin in 20 ml THF wurden unter Eiskühlung 0,86 g (6,7 mmol) Ethansulfonsäurechlorid zugetropft. Man ließ auf Raumtemperatur kommen, rührte über Nacht nach und destillierte das Lösungsmittel im Vakuum ab. Nach Verrühren des Rückstandes mit Wasser wurde das ausgefallene Produkt abgesaugt. Man erhielt 1,1 g 5-Ethylsulfonylamino-2,3,4,5-tetrahydro-1-benzoxepin. Fp 109 - 111 °C.
d) Zu einer Suspension von 0,16 g (5,4 mmol) 80proz. Natriumhydrid in 10 ml THF wurde unter Stickstoff eine Lösung von 1,0 g (3,9 mmol) 5-Ethylsulfonylamino-2,3,4,5-tetrahydro-1-benzoxepin in 15 ml THF zugetropft. Nach 3 h Rühren bei RT tropfte man 1,6 g (11 mmol) Methyliodid zu und ließ über Nacht bei RT weiterrühren. Nach Abdestillieren des Lösungsmittels wurde der Rückstand mit Wasser versetzt und mit EE extrahiert. Die organische Phase wurde nach Trocknung über Natriumsulfat im Vakuum eingeengt. Man erhielt 1,0 g 5-(N-Ethylsulfonyl-N-methylamino)-2,3,4,5-tetrahydro-1-benzoxepin. Fp 122 - 124 °C.

### Beispiel 7: 5-(N-Butyl-N-ethylsulfonyl-amino)-2,3,4,5-tetrahydro-1-benzoxepin

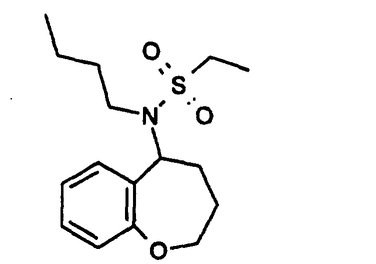

Aus 7,1 g 5-Ethylsulfonylamino-2,3,4,5-tetrahydro-1-benzoxepin (Beispiel 6 c) wurden durch Alkylierung mit Butyliodid analog Beispiel 11 8,3 g 5-(N-Butyl-N-ethylsulfonyl-amino)-2,3,4,5-tetrahydro-1-benzoxepin als viskoses Öl erhalten, das nach längerer Zeit kristallisierte. Fp 62 - 65°C.

### Beispiel 8: 5-(N-Methyl-N-methylsulfonyl-amino)-2,3,4,5-tetrahydro-1-benzoxepin

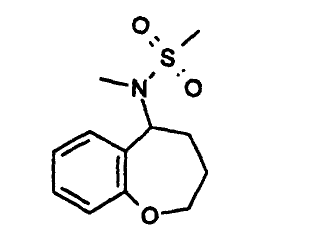

a) Zu einer Lösung von 2,0 g (12,2 mmol) 5-Amino-2,3,4,5-tetrahydro-1-benzoxepin (Beispiel 6 b) und 3,7 g (36,6 mmol) Triethylamin in 40 ml THF wurden unter Eiskühlung 1,54 g (13,4 mmol) Methansulfonsäurechlorid zugetropft. Man ließ auf RT kommen, rührte über Nacht nach, versetzte mit 50 ml Wasser und destillierte das THF im Vakuum ab. Der Rückstand wurde mit weiteren 50 ml Wasser verdünnt, 3 h gerührt und das ausgefallene Produkt abgesaugt. Nach Trocknen im Vakuum erhielt man 2,2 g 5-Methylsulfonylamino-2,3,4,5-tetrahydro-1-benzoxepin. Fp 105 - 106°C.
b) Eine Lösung von 1,1 g (6,2 mmol) 5-Methylsulfonylamino-2,3,4,5-tetrahydro-1-benzoxepin in 15 ml THF wurde zu einer Suspension von 0,23 g (6,2 mmol) 80proz. Natriumhydrid in 10 ml THF zugetropft. Nach 2 h bei RT wurden 0,94 g (6,7 mmol) lodmethan zugefügt und über Nacht bei RT nachgerührt. Nach Abdestillieren des Lösungsmittels im Vakuum wurde der Rückstand in EE aufgenommen, mit verd.
Salzsäure und Wasser gewaschen, über Magnesiumsulfat getrocknet und eingeengt. Anschließende Umkristallisation des Produktes aus Isopropanol lieferte 0,5 g 5-(N-Methyl-N-methylsulfonyl-amino)-2,3,4,5-tetrahydro-1-benzoxepin. Fp 143 - 145°C.

### Beispiel 9: 5-(N-Butyl-N-methylsulfonyl-amino)-2,3,4,5-tetrahydro-1-benzoxepin

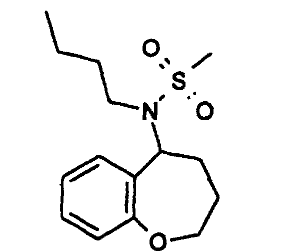

1,0 g 5-Methylsulfonylamino-2,3,4,5-tetrahydro-1-benzoxepin (Beispiel 8 a) wurden analog Beispiel 11 mit Natriumhydrid und lodbutan in DMF umgesetzt. Nach Umkristallisation des Rohproduktes (1,0 g) aus Isopropanol wurden 0,4 g 5-(N-Butyl-N-methylsulfonyl-amino)-2,3,4,5-tetrahydro-1-benzoxepin erhalten. Fp 78 - 79°C.

### Beispiel 10: 7-Chlor-9-methyl-5-(N-ethylsulfonyl-N-methyl-amino)-2,3,4,5-tetrahydro-1-benzoxepin

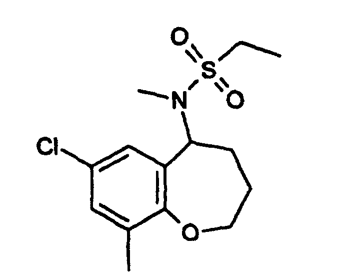

a) Eine Mischung aus 90 g 4-(4-Chlor-2-methylphenoxy)-buttersäure (Aldrich) und 1000 g Polyphosphorsäure wurde 4,5 h bei 85°C gerührt. Dann wurde der Ansatz auf 5 I Eiswasser gegossen, 1 h nachgerührt und mit Ether extrahiert. Die vereinigten organischen Phasen wurden mehrmals mit Sodalösung und Wasser gewaschen, über Magnesiumsulfat getrocknet und einrotiert. Der dunkle Rückstand wurde dann erneut in Ether aufgenommen und mehrmals mit Aktivkohle und Kieselgel aufgekocht, sowie abfiltriert, bis die Lösung nur noch schwach gefärbt war. Nach dem Einengen erhielt man 48,4 g 7-Chlor-9-methyl-3,4-dihydro-2H-1-benzoxepin-5-on; Fp.: 56 - 58°C.
b) 3,0 g 7-Chlor-9-methyl-3,4-dihydro-2H-1-benzoxepin-5-on, 50 ml wasserfreies Methanol, 10,9 g Ammoniumacetat und 0,63 g Natriumcyanborhydrid wurden 5 h bei 60°C und dann 2 Tage bei RT gerührt. Dann wurde die Reaktionsmischung mit Salzsäure angesäuert und am Rotationsverdampfer eingeengt. Der Rückstand wurde in Wasser aufgenommen, mit Ammoniaklösung alkalisch gestellt und mit Essigester extrahiert. Nach Trocknen und Einengen wurde das Produkt durch Chromatographie an Kieselgel mit Essigester/Methanol 9 : 1 gereinigt und man erhielt 1,3 g 5-Amino-7-chlor-9-methyl-2,3,4,5-tetrahydro-1-benzoxepin.
c) Zu einer Lösung von 1,3 g 5-Amino-7-chlor-9-methyl-2,3,4,5-tetrahydro-1-benzoxepin und 2,4 g Triethylamin in 30 ml THF wurden unter Eiskühlung 0,86 g Ethansulfonsäurechlorid zugetropft. Man ließ auf Raumtemperatur kommen, rührte über Nacht nach und destillierte das Lösungsmittel im Vakuum ab. Nach Verrühren des Rückstandes mit Wasser wurde das ausgefallene Produkt abgesaugt und im Vakuum getrocknet. Man erhielt 1,6 g 7-Chlor-9-methyl-5-(N-ethylsulfonylamino)-2,3,4,5-tetrahydro-1-benzoxepin; Fp. 144 - 145°C.
d) Zu einer Suspension von 0,1 g (2,7 mmol) 80 proz. Natriumhydrid in 5 ml THF wurde unter Stickstoff eine Lösung von 0,6 g (2,0 mmol) 7-Chlor-9-methyl-5-(Nethylsulfonylamino)-2,3,4,5-tetrahydro-1-benzoxepin in 8 ml THF zugetropft. Nach 1 h Rühren bei RT tropfte man 0,41 g (2,9 mmol) Methyliodid zu und ließ über Nacht bei RT weiterrühren. Nach Abdestillieren des Lösungsmittels wurde der Rückstand mit Wasser versetzt und mit EE extrahiert. Nach Waschen der organischen Phase mit verd. Salzsäure und Wasser, sowie Trocknen über Magnesiumsulfat wurde im Vakuum eingeengt und das Rohprodukt aus Methylenchlorid umkristallisiert. Man erhielt 0,4 g 7-Chlor-9-methyl-5-(N-ethylsulfonyl-N-methyl-amino)-2,3,4,5-tetrahydro-1-benzoxepin; Fp.: 141 - 143°C.
   ¹H-NMR (CDCl₃): δ [ppm] = 1.35 (3H), 1.9-2.2 (4H), 2.2 (3H), 2.9 (3H), 3.05 (2H), 3.7 (1H), 4.2 (1H), 5.15 (1 H), 7.1 (2H).

### Beispiel 11: 5-(N-Butyl-N-ethylsulfonyl-amino)-7-chlor-9-methyl-2,3,4,5-tetrahydro-1-benzoxepin

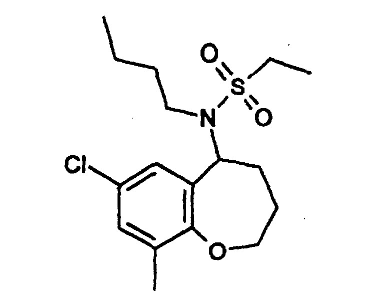

Zu einer Suspension von 0,1 g (2,7 mmol) 80 proz. Natriumhydrid in 8 ml DMF wurde unter Stickstoff eine Lösung von 0,8 g (2,6 mmol) 7-Chlor-9-methyl-5-(Nethylsulfonylamino)-2,3,4,5-tetrahydro-1-benzoxepin (Beispiel 10 c) in 10 ml DMF zugetropft. Nach 30 min Rühren bei RT tropfte man 0,68 g (3,7 mmol) Butyliodid zu und ließ über Nacht bei RT weiterrühren. Nach Abdestillieren des Lösungsmittels wurde der Rückstand mit Wasser versetzt und mit EE extrahiert. Nach Waschen der organischen Phase mit verd. Salzsäure und Wasser, sowie Trocknen über Magnesiumsulfat wurde im Vakuum eingeengt. Man erhielt 0,9 g 5-(N-Butyl-N-ethylsulfonyl-amino)-7-Chlor-9-methyl-2,3,4,5-tetrahydro-1-benzoxepin; Fp. 83 - 87°C.

### Beispiel 12: 5-(N-Butyl-N-methylsulfonyl-amino)-7-Chlor-9-methyl-2,3,4,5-tetrahydro-1-benzoxepin

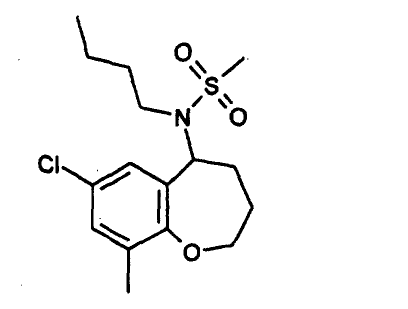

0,6 g 5-Amino-7-chlor-9-methyl-2,3,4,5-tetrahydro-1-benzoxepin (Beispiel 10 b) wurden analog Beispiel 10 c mit Methansulfonsäurechlorid umgesetzt. Man erhielt 0,6 g 7-Chlor-9-methyl-5-methylsulfonylamino-2,3,4,5-tetrahydro-1-benzoxepin; Fp.: 154 - 156°C. Durch anschließende Alkylierung mit Butyliodid analog Beispiel 11 erhielt man 0,6 g 5-(N-Butyl-N-methylsulfonyl-amino)-7-Chlor-9-methyl-2,3,4,5-tetrahydro-1-benzoxepin; Fp.: 106 - 110°C.

### Beispiel 13: 5-(N-Butyl-N-methylsulfonyl-amino)-7-nitro-2,3,4,5-tetrahydro-1-benzoxepin

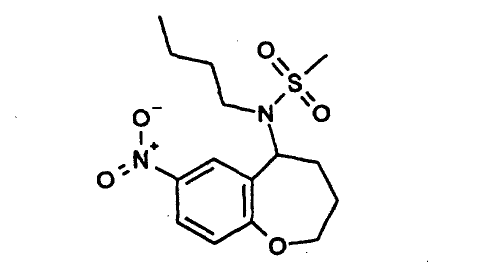

a) 10,0 g (61,7 mmol) 3,4-Dihydro-1-benzoxepin-5(2H)-on (J. Chem. Soc., Perkin Trans. 1, 1991, 2763) wurden unter Eiskühlung in 80 ml konz. Schwefelsäure eingetragen. Dann wurden 5,77 g (67,9 mmol) Natriumnitrat zugegeben und 90 min bei 0°C gerührt. Die Reaktionsmischung wurde auf 800 ml Eiswasser gegossen, das ausgefallene Produkt wurde abgesaugt, neutral gewaschen und im Vakuum getrocknet. Nach Umkristallisation aus Isopropanol erhielt man 7 g 3,4-Dihydro-7-nitro-1-benzoxepin-5(2H)-on, das mit ca. 12% der entsprechenden 7,9-Dinitroverbindung verunreinigt war. Fp 112 - 116°C.
b) 3,4 g (16,4 mmol) 3,4-Dihydro-7-nitro-1-benzoxepin-5(2H)-on wurden mit 12,7 g (164 mmol) Ammoniumacetat und 7,2 g (115 mmol) Natriumcyanborhydrid in 55 ml Methanol 3 h bei 60°C gerührt. Nach Zugabe von 10 ml Wasser wurde der Ansatz im Vakuum eingeengt und der Rückstand in Essigester aufgenommen und mit verd. Natronlauge gewaschen. Die EE-Phase wurde mit verd. Salzsäure extrahiert und die erhaltene Salzsäurephase wurde mit Natronlauge alkalisch gestellt und mit EE extrahiert. Nach Trocknen des EE-Extraktes wurden 1,7 g 5-Amino-7-nitro-2,3,4,5-tetrahydro-1-benzoxepin erhalten.
c) Aus 1,6 g 5-Amino-7-nitro-2,3,4,5-tetrahydro-1-benzoxepin wurden analog Beispiel 8 a 1,9 g 5-Methylsulfonylamino-7-nitro-2,3,4,5-tetrahydro-1-benzoxepin erhalten. Fp 150 - 151°C.
d) Aus 0,5 g 5-Methylsulfonylamino-7-nitro-2,3,4,5-tetrahydro-1-benzoxepin wurden durch Umsetzung mit Natriumhydrid und lodbutan in DMF analog Beispiel 11 0,6 g 5-(N-Butyl-N-methylsulfonyl-amino)-7-nitro-2,3,4,5-tetrahydro-1-benzoxepin erhalten. Fp 96 - 98°C.

### Beispiel 14: 7-Butoxy-3,4-dihydro-2H-1-benzoxepin-5-on

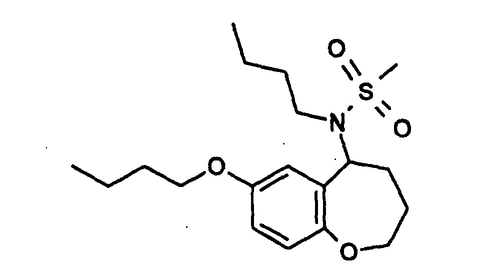

a) Aus 4-Butoxyphenol kann durch Alkylierung mit 4-Brombuttersäureethylester, gefolgt von Verseifung und Cyclisierung in Gegenwart von Polyphosphorsäure 7-Butoxy-3,4-dihydro-2H-1-benzoxepin-5-on erhalten werden, wie in J. Heterocyclic Chem. 26, 1989, 1547 für das analoge 7-Propoxy-3,4-dihydro-2H-1-benzoxepin-5-on beschrieben.
b) Aus 7-Butoxy-3,4-dihydro-2H-1-benzoxepin-5-on kann durch reduktive Aminierung mit Ammoniumacetat und Natriumcyanborhydrid gefolgt von Umsetzung mit Methansulfonsäurechlorid und schließlich Alkylierung mit Butyliodid, wie in Beispiel 13 b - d beschrieben, 7-Butoxy-3,4-dihydro-2H-1-benzoxepin-5-on erhalten werden.

### Pharmakologische Untersuchungen

I_{sK}-Kanäle aus Mensch, Ratte oder Meerschweinchen wurden in Xenopus-Oozyten exprimiert. Hierfür wurden zuerst Oozyten aus Xenopus Laevis isoliert und defollikuliert. Anschließend wurde in diese Oozyten in vitro synthetisierte I_{sK}kodierende RNA injiziert. Nach 2 - 8 Tagen I_{sK}-Proteinexpression wurden an den Oozyten mit der Zwei-Mikroelektroden Voltage-Clamp Technik I_{sK}-Ströme gemessen. Die I_{sK}-Kanäle wurden hierbei in der Regel mit 15 s dauernden Spannungssprüngen auf -10 mV aktiviert. Das Bad wurde mit einer Lösung der nachfolgenden Zusammensetzung durchspült: NaCl 96 mM, KCI 2 mM, CaCl₂ 1,8 mM, MgCl₂ 1 mM, HEPES 5 mM (titriert mit NaOH auf pH 7,5). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des I_{sK}-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

### Literatur:

A.E. Busch, H.-G. Kopp, S. Waldegger, I. Samarzija, H. Süßbrich, G. Raber, K.

Kunzelmann, J. P. Ruppersberg und F. Lang; "Inhibition of both exogenously expressed I_{sK} and endogenous K⁺ channels in Xenopus oocytes by isosorbide dinitrate"; J. Physiol. 491 (1995), 735-741;
T. Takumi, H. Ohkubo und S. Nakanishi; "Cloning of a membrane protein that induces a slow voltage-gated potassium current"; Science 242 (1989), 1042-1045; M. D. Vamum, A.E. Busch, C.T. Bond, J. Maylie und J.P. Adelman; "The minK channel underlies the cardiac potassium current and mediates species-specific responses to protein kinase"; C. Proc. Natl. Acad. Sci. USA 90 (1993), 11528-11532.

Auf die beschriebene Weise wurden für die Verbindung des Beispiels 13 ein IC₅₀-Wert von 3.3 µmol / I ermittelt.

## Patentansprüche

1. Verbindungen der Formel I, 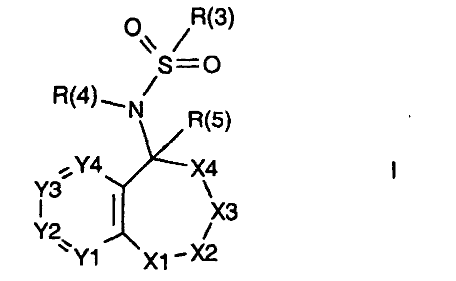 worin bedeuten
X1 -O- oder -CR(1)R(2)-;
R(1) und R(2)
unabhängig voneinander Wasserstoff, CF₃, Alkyl mit 1, 2, 3, 4, 5 oder 6 C-Atomen;
X2 -CR(1 )R(2)-,
wobei die Reste R(1) und R(2)
wie bei X1 angegeben definiert sind, aber unabhängig von den Bedeutungen der Reste in X1 sind;
X3 -CR(1)R(2)-,
wobei die Reste R(1) und R(2)
wie bei X1 angegeben definiert sind, aber unabhängig von den Bedeutungen der Reste in X1 sind;
X4 -CR(1 )R(2)- oder -CH(OR(30))-;
wobei die Reste R(1) und R(2)
wie bei X1 angegeben definiert sind, aber unabhängig von den Bedeutungen der Reste in X1 sind;
R(30) Wasserstoff, Alkyl mit 1, 2 oder 3 C-Atomen oder Acyl mit 1, 2, 3 oder 4 C-Atomen;
Y1, Y2, Y3 und Y4 unabhängig voneinander -CR(12)-;
die Reste R(12)
unabhängig voneinander Wasserstoff, F, Cl, Br, I, Alkyl mit 1, 2, 3, 4 oder 5 C-Atomen, CN, CF₃, NO₂, -Z-CₘH₂ₘ-R(13) oder Phenyl,
das unsubstituiert ist oder substituiert ist mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Z -O-, -CO-, -CO-O-, -O-CO-, -S-, -SO-, -SO₂-, -SO₂NR(14)-, - NR(14)- oder -CONR(14)-;
R(14) Wasserstoff oder Alkyl mit 1, 2 oder 3 C-Atomen;
m Null, 1, 2 oder 3;
R(13) Wasserstoff, CF₃, -NR(15)R(16), -CONR(15)R(16), -OR(30a), Phenyl, Thienyl oder einen N-haltigen Heterocyclus mit 1, 2, 3, 4, 5, 6, 7, 8 oder 9 C-Atomen,
wobei Phenyl, Thienyl oder der N-haltige Heterocyclus unsubstituiert sind oder substituiert sind mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16)
unabhängig voneinander Wasserstoff oder Alkyl mit 1, 2 oder 3 C-Atomen; oder
R(15) und R(16)
gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(30a)
Wasserstoff, Alkyl mit 1, 2 oder 3 C-Atomen oder Acyl mit 1, 2, 3 oder 4 C-Atomen;
R(3) R(17)-CₓH₂ₓ-,
R(17) Methyl, Cycloalkyl mit 3, 4, 5 oder 6 C-Atomen oder CF₃;
x Null, 1, 2, 3, 4, 5, 6, 7, 8, 9 oder 10;
R(4) -CᵣH₂ᵣ-R(20),
wobei eine CH₂-Gruppe der Gruppe CᵣH₂ᵣ ersetzt sein kann durch -O-, -CO-O, -O-CO-, -NR(21)- oder -CONR(21)-;
R(21) Wasserstoff oder Alkyl mit 1, 2 oder 3 C-Atomen;
r Null, 1, 2, 3, 4, 5, 6, 7, 8, 9 oder 10;
R(20) Methyl, CF₃ oder Cycloalkyl mit 3, 4, 5 oder 6 C-Atomen;
R(5) Wasserstoff;
in allen ihren stereoisomeren Formen und Gemische davon in beliebigen Verhältnissen, sowie ihre physiologisch verträglichen Salze.

2. Verbindungen der Formel I nach Anspruch 1, worin bedeuten:
X1 -O- oder -CH₂;
X2 -CR(1)R(2)-,
R(1) und R(2)
unabhängig voneinander Wasserstoff oder Alkyl mit 1, 2 oder 3 C-Atomen;
X3 -CH₂- oder -C(CH₃)₂-;
X4 -CH₂- oder -CHOH-;
Y1, Y2, Y3 und Y4 unabhängig voneinander -CR(12)-;
die Reste R(12)
unabhängig voneinander Wasserstoff, F, Cl, Br, Alkyl mit 1, 2 oder 3 C-Atomen, CN, CF₃, NO₂, -Z-CₘH₂ₘ-R(13);
Z -O-, -CO-, -CO-O-, -O-CO-, -S-, -SO-, -SO₂-, -SO₂NR(14)-, -NR(14)- oder -CONR(14)-;
R(14) Wasserstoff oder Alkyl mit 1, 2 oder 3 C-Atomen;
m Null, 1, 2 oder 3;
R(13) Wasserstoff, CF₃, -NR(15)R(16), Phenyl, Piperidyl, 1-Pyrrolidinyl, 4-Morpholinyl, 4-Methylpiperazin-1-yl, Pyridyl, Thienyl oder Imidazolyl;
R(15) und R(16)
unabhängig voneinander Wasserstoff oder Alkyl mit 1, 2 oder 3 C-Atomen;
R(3) R(17)-CₓH₂ₓ-,
R(17) Methyl;
x Null, 1, 2 oder 3;
R(4) -CᵣH₂ᵣ-R(20), wobei eine CH₂-Gruppe der Gruppe CᵣH₂ᵣ ersetzt sein kann durch -O-, -CO-O-, -O-CO-, -NR(21)- oder -CONR(21)-;
R(21) Wasserstoff oder Alkyl mit 1, 2 oder 3 C-Atomen;
r Null, 1, 2, 3, 4, 5 oder 6;
R(20) Methyl, CF₃ oder Pyridyl;
R(5) Wasserstoff;
in allen ihren stereoisomeren Formen und Gemische davon in beliebigen Verhältnissen, sowie ihre physiologisch verträglichen Salze.

3. Verbindungen der Formel I nach Anspruch 1 oder 2, worin bedeuten:
X1 -O-;
X2 -CR(1)R(2)-,
R(1) und R(2)
unabhängig voneinander Wasserstoff, Alkyl mit 1, 2 oder 3 C-Atomen;
X3 -CH₂- oder -C(CH₃)₂;
X4 -CH₂-;
Y1 CH;
Y2 CH;
Y4 CH;
Y3 -CR(12)-;
R(12)
F, Cl, Br, Alkyl mit 1, 2 oder 3 C-Atomen, CN, CF₃, NO₂, -Z-CₘH₂ₘ-R(13);
Z -O-, -CO-, -CO-O-, -O-CO-, -S-, -SO-, -SO₂-, -SO₂NR(14)- oder -CONR(14)-;
R(14) Wasserstoff oder Alkyl mit 1, 2 oder 3 C-Atomen;
m 1, 2 oder 3;
R(13) Wasserstoff, CF₃, Pyridyl oder Phenyl;
R(3) R(17)-CₓH₂ₓ-,
R(17) Methyl;
x Null, 1 oder 2;
R(4) -CᵣH₂ᵣ-R(20),
wobei eine CH₂-Gruppe der Gruppe CᵣH₂ᵣ ersetzt sein kann durch -O-, -CO-O-, -O-CO- oder -CONR(21)-;
R(21) Wasserstoff oder Alkyl mit 1, 2 oder 3 C-Atomen;
r Null, 1, 2, 3, 4, 5 oder 6;
R(20) Methyl oder CF₃;
R(5) Wasserstoff;
in allen ihren stereoisomeren Formen und Gemischen davon in beliebigen Verhältnissen, sowie ihre physiologisch verträglichen Salze.

4. Verbindungen der Formel I gemäß einem oder mehreren der Ansprüche 1 bis 3 und ihre physiologisch verträglichen Salze zur Anwendung als Arzneimittel.

5. Pharmazeutische Zubereitung, enthaltend eine wirksame Menge mindestens einer Verbindung der Formel I nach einem oder mehreren der Ansprüche 1 bis 3 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmazeutischen Wirkstoffen.

6. Verwendung einer Verbindung der Formel I nach einem oder mehreren der Ansprüche 1 bis 3 und/oder eines physiologisch verträglichen Salzes davon zur Herstellung eines Medikaments mit K⁺-Kanal-blockierender Wirkung zur Therapie und Prophylaxe von K⁺-Kanal-mediierten Krankheiten.

7. Verwendung einer Verbindung der Formel I nach einem oder mehreren der Ansprüche 1 bis 3 und/oder eines physiologisch verträglichen Salzes davon zur Herstellung eines Medikaments zum Inhibieren der Magensäuresekretion.

8. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe von Ulcera des Magens oder des intestinalen Bereiches.

9. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe der Refluxösophagitis.

10. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe von Durchfallerkrankungen.

11. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

12. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

13. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe von atrialer Fibrillation oder atrialem Flattern.

14. Verwendung einer Verbindung der Formel I nach einem oder mehreren der Ansprüche 1 bis 3 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmern.

15. Verwendung einer Verbindung der Formel I nach einem oder mehreren der Ansprüche 1 bis 3 und/oder eines physiologisch verträglichen Salzes davon zur Herstellung eines Medikaments zur Therapie der Herzinsuffizienz.

## Claims

1. A compound of the formula I, 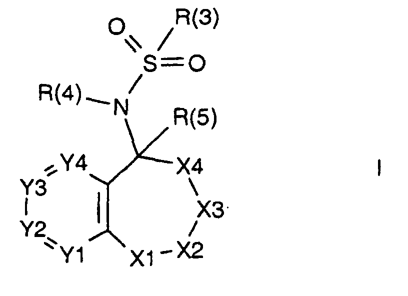 in which:
X1 is -O- or -CR(1 )R(2)-;
R(1) and R(2)
independently of one another are hydrogen, CF₃, alkyl having 1, 2, 3, 4, 5 or 6 carbon atoms;
X2 is -CR(1)R(2)-,
where the radicals R(1) and R(2)
are defined as indicated for X1, but are independent of the meanings of the radicals in X1;
X3 is -CR(1 )R(2)-,
where the radicals R(1) and R(2)
are defined as indicated for X1, but are independent of the
meanings of the radicals in X1;
X4 is -CR(1)R(2)- or -CH(OR(30))-;
where the radicals R(1) and R(2)
are defined as indicated for X1, but are independent of the meanings of the radicals in X1;
R(30) is hydrogen, alkyl having 1, 2 or 3 carbon atoms or acyl having 1, 2, 3 or 4 carbon atoms;
Y1, Y2, Y3 and Y4 independently of one another are -CR(12)-;
the radicals R(12)
independently of one another are hydrogen, F, Cl, Br, I, alkyl having 1, 2, 3, 4 or 5 carbon atoms, CN, CF₃, NO₂, -Z-CₘH₂ₘ-R(13) or phenyl,
which is unsubstituted or is substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Z is -O-, -CO-, -CO-O-, -O-CO-, -S-, -SO-, -SO₂-, -SO₂NR(14)-, -NR(14)- or -CONR(14)-;
R(14) is hydrogen or alkyl having 1, 2 or 3 carbon atoms;
m is zero, 1, 2 or 3;
R(13) is hydrogen, CF₃, -NR(15)R(16), -CONR(15)R(16), -OR(30a), phenyl, thienyl or an N-containing heterocycle having 1, 2, 3, 4, 5, 6, 7, 8 or 9 carbon atoms,
where phenyl, thienyl or the N-containing heterocycle are unsubstituted or are substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16)
independently of one another are hydrogen or alkyl having 1, 2 or 3 carbon atoms;
or
R(15) and R(16)
together are a chain of 4 or 5 methylene groups, of which a CH₂ group can be replaced by -O-, -S-, -NH-, -N(CH₃)- or -N(benzyl)-;
R(30a)
is hydrogen, alkyl having 1, 2 or 3 carbon atoms or acyl having 1, 2, 3 or 4 carbon atoms;
R(3) is R(17)-CₓH₂ₓ-,
R(17) is methyl, cycloalkyl having 3, 4, 5 or 6 carbon atoms or CF₃;
x is zero, 1, 2, 3, 4, 5, 6, 7, 8, 9 or 10;
R(4) is -CᵣH₂ᵣ-R(20),
where a CH₂ group of the group CᵣH₂ᵣ can be replaced by -O-, -CO-O, -O-CO-, -NR(21)- or -CONR(21)-;
R(21) is hydrogen or alkyl having 1, 2 or 3 carbon atoms;
r is zero, 1, 2, 3, 4, 5, 6, 7, 8, 9 or 10;
R(20) is methyl, CF₃ or cycloalkyl having 3, 4, 5 or 6 carbon atoms;
R(5) is hydrogen;
in all its stereoisomeric forms and mixtures thereof in any desired ratios, or its physiologically tolerable salts.

2. A compound of the formula I as claimed in claim 1, in which:
X1 is -O- or -CH₂;
X2 is -CR(1)R(2)-,
R(1) and R(2)
independently of one another are hydrogen or alkyl having 1, 2 or 3 carbon atoms;
X3 is -CH₂- or -C(CH₃)₂-;
X4 is -CH₂- or -CHOH-;
Y1, Y2, Y3 and Y4 independently of one another are -CR(12)-; the radicals R(12)
independently of one another are hydrogen, F, Cl, Br, alkyl having 1, 2 or 3 carbon atoms, CN, CF₃, NO₂, -Z-CₘH₂ₘ-R(13);
Z is -O-, -CO-, -CO-O-, -O-CO-, -S-, -SO-, -SO₂-, -SO₂NR(14)-, -NR(14)- or -CONR(14)-;
R(14) is hydrogen or alkyl having 1, 2 or 3 carbon atoms;
m is zero, 1, 2 or 3;
R(13) is hydrogen, CF₃, -NR(15)R(16), phenyl, piperidyl, 1-pyrrolidinyl, 4-morpholinyl, 4-methylpiperazin-1-yl, pyridyl, thienyl or imidazolyl;
R(15) and R(16)
independently of one another are hydrogen or alkyl having 1, 2 or 3 carbon atoms;
R(3) is R(17)-CₓH₂ₓ-,
R(17) is methyl;
x is zero, 1, 2 or 3;
R(4) is -CᵣH₂ᵣ-R(20),
where a CH₂ group of the group CᵣH₂ᵣ can be replaced by -O-, -CO-O-, -O-CO-, -NR(21)- or -CONR(21)-;
R(21) is hydrogen or alkyl having 1, 2 or 3 carbon atoms;
r is zero, 1, 2, 3, 4, 5 or 6;
R(20) is methyl, CF₃ or pyridyl;
R(5) is hydrogen;
in all its stereoisomeric forms and mixtures thereof in any desired ratios, or its physiologically tolerable salts.

3. A compound of the formula I as claimed in claim 1 or 2, in which:
X1 is -O-;
X2 is -CR(1)R(2)-,
R(1) and R(2)
independently of one another are hydrogen or alkyl having 1, 2 or 3 carbon atoms;
X3 is -CH₂ or -C(CH₃)₂;
X4 is -CH₂-;
Y1 is CH;
Y2 is CH;
Y4 is CH;
Y3 is -CR(12)-;
R(12)
is F, Cl, Br, alkyl having 1, 2 or 3 carbon atoms, CN, CF₃, NO₂, -Z-CₘH₂ₘ-R(13);
Z is -O-, -CO-, -CO-O-, -O-CO-, -S-, -SO-, -SO₂-, -SO₂NR(14)- or -CONR(14)-;
R(14) is hydrogen or alkyl having 1, 2 or 3 carbon atoms;
m is 1, 2 or 3;
R(13) is hydrogen, CF₃, pyridyl or phenyl;
R(3) is R(17)-CₓH₂ₓ-,
R(17) is methyl;
x is zero, 1 or 2;
R(4) is -CᵣH₂ᵣ-R(20),
where a CH₂ group of the group CᵣH₂ᵣ can be replaced by -O-, -CO-O-, -O-CO- or -CONR(21)-;
R(21) is hydrogen or alkyl having 1, 2 or 3 carbon atoms;
r is zero, 1, 2, 3, 4, 5 or 6;
R(20) is methyl or CF₃;
R(5) is hydrogen;
in all its stereoisomeric forms and mixtures thereof in any desired ratios, or its physiologically tolerable salts.

4. A compound of the formula I as claimed in one or more of claims 1 to 3 and its physiologically tolerable salts for use as a pharmaceutical.

5. A pharmaceutical preparation comprising an effective amount of at least one compound of the formula I as claimed in one or more of claims 1 to 3 and/or a physiologically tolerable salt thereof as active compound, together with pharmaceutically acceptable excipients and additives and, if appropriate, additionally one or more other pharmaceutical active compounds.

6. The use of a compound of the formula I as claimed in one or more of claims 1 to 3 and/or of a physiologically tolerable salt thereof for the production of a medicament with K⁺ channel-blocking action for the therapy and prophylaxis of K⁺ channel-mediated illnesses.

7. The use of a compound of the formula I as claimed in one or more of claims 1 to 3 and/or of a physiologically tolerable salt thereof for the production of a medicament for inhibiting gastric acid secretion.

8. The use of a compound of the formula I as claimed in one or more of claims 1 to 3 and/or of a physiologically tolerable salt thereof for the production of a medicament for the therapy or prophylaxis of ulcers of the stomach or of the intestinal region.

9. The use of a compound of the formula I as claimed in one or more of claims 1 to 3 and/or of a physiologically tolerable salt thereof for the production of a medicament for the therapy or prophylaxis of reflux esophagitis.

10. The use of a compound of the formula I as claimed in one or more of claims 1 to 3 and/or of a physiologically tolerable salt thereof for the production of a medicament for the therapy or prophylaxis of diarrheal disorders.

11. The use of a compound of the 'formula I as claimed in one or more of claims 1 to 3 and/or of a physiologically tolerable salt thereof for the production of a medicament for the therapy or prophylaxis of all types of arrhythmias, including atrial, ventricular and supraventricular arrhythmias.

12. The use of a compound of the formula I as claimed in one or more of claims 1 to 3 and/or of a physiologically tolerable salt thereof for the production of a medicament for the therapy or prophylaxis of cardiac arrhythmias which can be eliminated by action potential prolongation.

13. The use of a compound of the formula I as claimed in one or more of claims 1 to 3 and/or of a physiologically tolerable salt thereof for the production of a medicament for the therapy or prophylaxis of atrial fibrillation or atrial flutter.

14. The use of a compound of the formula I as claimed in one or more of claims 1 to 3 and/or of a physiologically tolerable salt thereof for the production of a medicament for the therapy or prophylaxis of reentry arrhythmias or for the prevention of sudden heart death as a result of ventricular fibrillation.

15. The use of a compound of the formula I as claimed in one or more of claims 1 to 3 and/or of a physiologically tolerable salt thereof for the production of a medicament for the therapy of cardiac insufficiency.

## Revendications

1. Composés de formule I 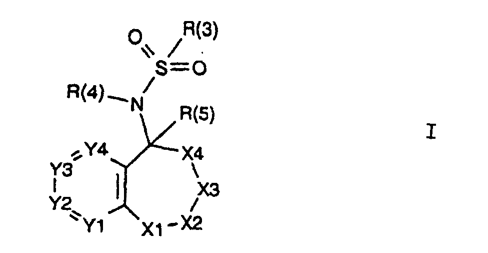 dans laquelle
X1 représente -O- ou -CR(1)R(2)- ;
R(1) et R(2)
représentent indépendamment l'un de l'autre un atome d'hydrogène, un groupe CF₃, alkyle ayant 1, 2, 3, 4, 5, 6 atomes de carbone ;
X2 représente -CR(1)R(2)-,
les radicaux R(1) et R(2)
étant définis comme indiqué à propos de X1, mais étant indépendants des significations des radicaux dans X1 ;
X3 représente -CR(1)R(2)-,
les radicaux R(1) et R(2)
étant définis comme indiqué à propos de X1, mais étant indépendants des significations des radicaux dans X1 ;
X4 représente -CR(1)R(2)- ou -CH(OR(30))-, les radicaux R(1) et R(2) étant définis comme indiqué à propos de X1, mais étant indépendants des significations des radicaux dans X1 ;
R(30) représente un atome d'hydrogène, un groupe alkyle ayant 1, 2 ou 3 atomes de carbone ou un groupe acyle ayant 1, 2, 3 ou 4 atomes de carbone ;
Y1, Y2, Y3 et Y4 représentent indépendamment les uns des autres -CR(12)- ;
les radicaux R(12)
représentent indépendamment les uns des autres un atome d'hydrogène, F, Cl, Br, I, un groupe alkyle ayant 1, 2, 3, 4 ou 5 atomes de carbone, CN, CF₃, NO₂, -Z- CₘH₂ₘ-R(13) ou un groupe phényle
qui est non substitué ou porte 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthyl-sulfonylamino ;
Z représente -O-, -CO-, -CO-O-, -O- CO-, -S-, -SO-, -SO₂-, -SO₂NR(14)-, -NR(14)- ou -CONR(14)- ;
R(14) représente un atome d'hydrogène ou un groupe alkyle ayant 1, 2 ou 3 atomes de carbone ;
m est égal à zéro, 1, 2 ou 3 ; R(13) représente un atome d'hydrogène, CF₃, -NR(15)R(16), -CONR(15)R(16), -OR(30a), un groupe phényle, thiényle ou un hétérocycle azoté ayant 1, 2, 3, 4, 5, 6, 7, 8 ou 9 atomes de carbone,
les groupes phényle, thiényle ou l'hétérocycle azoté étant non substitués ou portant 1 ou 2 substituants choisis dans l'ensemble constitué par F, Cl, Br, I, CF₃, NO₂, CN, NH₂, OH, les groupes méthyle, éthyle, méthoxy, diméthylamino, sulfamoyle, méthylsulfonyle et méthylsulfonylamino ;
R(15) et R(16) représentent indépendamment l'un de l'autre un atome d'hydrogène ou un groupe alkyle ayant 1, 2 ou 3 atomes de carbone ;
ou
R(15) et R(16) forment ensemble une chaîne de 4 ou 5 groupes méthylène, dont un groupe CH₂ peut être remplacé par -O-, -S-, -NH-, -N(CH₃)- ou -N(benzyle)- ;
R(30a) représente un atome d'hydrogène, un groupe alkyle ayant 1, 2 ou 3 atomes de carbone ou acyle ayant 1, 2, 3 ou 4 atomes de carbone ;
R(3) représente R(17)-CₓH₂ₓ-,
R(17) représente un groupe méthyle, cycloalkyle ayant 3, 4, 5 ou 6 atomes de carbone ou CF₃ ;
x est zéro, 1, 2, 3, 4, 5, 6, 7, 8, 9 ou 10 ;
R(4) représente -CᵣH₂ᵣ-R(20), un groupe CH₂ du groupe CᵣH₂ᵣ pouvant être remplacé par -O-, -CO-O-, -O-CO-, -NR(21)- ou -CONR(21)- ;
R(21) représente un atome d'hydrogène ou un groupe alkyle ayant 1, 2 ou 3 atomes de carbone ;
r est zéro, 1, 2, 3, 4, 5, 6, 7, 8, 9 ou 10 ;
R(20) représente un groupe méthyle, CF₃ ou cycloalkyle ayant 3, 4, 5 ou 6 atomes de carbone ;
R(5) représente un atome d'hydrogène ;
sous toutes leurs formes stéréoisomères et mélanges de celles-ci en rapports quelconques, ainsi que leurs sels physiologiquement acceptables.

2. Composés de formule I selon la revendication 1, dans lesquels
X1 représente -O- ou -CH₂ ;
X2 représente -CR(1)R(2)-,
R(1) et R(2)
représentent indépendamment l'un de l'autre un atome d'hydrogène ou un groupe alkyle ayant 1, 2 ou 3 atomes de carbone ;
X3 représente -CH₂- ou -C(CH₃)₂- ;
X4 représente -CH₂- ou -CHOH- ;
Y1, Y2, Y3 et Y4 représentent indépendamment les uns des autres -CR(12)- ;
les radicaux R(12)
représentent indépendamment les uns des autres un atome d'hydrogène, F, Cl, Br, un groupe alkyle ayant 1, 2 ou 3 atomes de carbone, CN, CF₃, NO₂, -Z-CₘH₂ₘ-R(13) ; Z représente -O-, -CO-, -CO-O-, -O- CO-, -S-, -SO-, -SO₂-, -SO₂NR(14)-, -NR(14)- ou -CONR(14)- ;
R(14) représente un atome d'hydrogène ou un groupe alkyle ayant 1, 2 ou 3 atomes de carbone ;
m est zéro, 1, 2 ou 3 ;
R(13) représente un atome d' hydrogène, CF₃, -NR(15)R(16), un groupe phényle, pipéridyle, 1-pyrrolidinyle, 4-morpholinyle, 4-méthylpipérazin-1-yle, pyridyle, thiényle ou imidazolyle ;
R(15) et R(16)
représentent indépendamment l'un de l'autre un atome d'hydrogène ou un groupe alkyle ayant 1, 2 ou 3 atomes de carbone, ;
R(3) représente R(17)-CₓH₂ₓ-,
R(17) représente le groupe méthyle ;
x est zéro, 1, 2 ou 3 ;
R(4) représente -CᵣH₂ᵣ-R(20),
un groupe CH₂ du groupe CᵣH₂ᵣ pouvant être remplacé par -O-, -CO-O-, -O-CO-, NR(21)- ou -CONR(21)- ;
R(21) représente un atome d'hydrogène ou un groupe alkyle ayant 1, 2 ou 3 atomes de carbone ;
r est zéro, 1, 2, 3, 4, 5 ou 6 ;
R(20) représente le groupe méthyle, CF₃ ou pyridyle ;
R(5) représente un atome d'hydrogène ;
sous toutes leurs formes stéréoisomères et mélanges de celles-ci en rapports quelconques, ainsi que leurs sels physiologiquement acceptables.

3. Composés de formule I selon la revendication 1 ou 2, dans lesquels
X1 représente -O- ;
X2 représente -CR(1)R(2)-,
R(1) et R(2)
représentent indépendamment l'un de l'autre un atome d'hydrogène ou un groupe alkyle ayant 1, 2 ou 3 atomes de carbone ;
X3 représente -CH₂- ou -C(CH₃)₂- ;
X4 représente -CH₂- ;
Y1 représente CH ;
Y2 représente CH ;
Y4 représente CH ;
Y3 représente -CR(12)- ;
R(12) représente
F, Cl, Br, un groupe alkyle ayant 1, 2 ou 3 atomes de carbone, CN, CF₃, NO₂, -Z-CₘH₂ₘ-R(13) ;
Z représente -O-, -CO-, -CO-O-, -O- CO-, -S-, -SO-, -SO₂-, -SO₂NR(14)- ou -CONR(14)- ;
R(14) représente un atome d'hydrogène ou un groupe alkyle ayant 1, 2 ou 3 atomes de carbone ;
m est 1, 2 ou 3 ;
R(13) représente un atome d'hydrogène, un groupe CF₃, pyridine ou phényle ;
R(3) représente R(17)-CₓH₂ₓ-,
R(17) représente le groupe méthyle ;
x est zéro, 1 ou 2 ;
R(4) représente -CᵣH₂ᵣ-R(20),
un groupe CH₂ du groupe CᵣH₂ᵣ pouvant être remplacé par -CO-O-, -O-CO- ou -CONR(21)- ;
R(21) représente un atome d'hydrogène ou un groupe alkyle ayant 1, 2 ou 3 atomes de carbone ;
r est zéro, 1, 2, 3, 4, 5 ou 6 ;
R(20) représente le groupe méthyle ou
R(5) représente un atome d'hydrogène ;
sous toutes leurs formes stéréoisomères et mélanges de celles-ci en rapports quelconques, ainsi que leurs sels physiologiquement acceptables.

4. Composés de formule I selon une ou plusieurs des revendications 1 à 3, et leurs sels physiologiquement acceptables, pour utilisation en tant que médicament.

5. Préparation pharmaceutique contenant une quantité efficace d'au moins un composé de formule I selon une ou plusieurs des revendications 1 à 3 et/ou d'un sel physiologiquement acceptable d'un tel composé, en tant que substance active, conjointement avec des véhicules et additifs pharmaceutiquement acceptables et éventuellement encore une ou plusieurs autres substances actives pharmaceutiques.

6. Utilisation d'un composé de formule I selon une ou plusieurs des revendications 1 à 3 et/ou d'un sel physiologiquement acceptable d'un tel composé, pour la fabrication d'un médicament à action bloquant les canaux K⁺, pour la thérapie et la prophylaxie de maladies médiées par les canaux K⁺.

7. Utilisation d'un composé de formule I selon une ou plusieurs des revendications 1 à 3 et/ou d'un sel physiologiquement acceptable d'un tel composé, pour la fabrication d'un médicament destiné à l'inhibition de la sécrétion d'acide gastrique.

8. Utilisation d'un composé de formule I selon une ou plusieurs des revendications 1 à 3 et/ou d'un sel physiologiquement acceptable d'un tel composé, pour la fabrication d'un médicament destinée à la thérapie ou à la prophylaxie d'ulcères de l'estomac ou de la région intestinale.

9. Utilisation d'un composé de formule I selon une ou plusieurs des revendications 1 à 3 et/ou d'un sel physiologiquement acceptable d'un tel composé, pour la fabrication d'un médicament destiné à la thérapie ou à la prophylaxie du reflux oesophagien.

10. Utilisation d'un composé de formule I selon une ou plusieurs des revendications 1 à 3 et/ou d'un sel physiologiquement acceptable d'un tel composé, pour la fabrication d'un médicament destiné à la thérapie ou à la prophylaxie de maladies diarrhéiques.

11. Utilisation d'un composé de formule I selon une ou plusieurs des revendications 1 à 3 et/ou d'un sel physiologiquement acceptable d'un tel composé, pour la fabrication d'un médicament destiné à la thérapie ou à la prophylaxie de tous types d'arythmies, y compris des arythmies auriculaires, ventriculaires et supraventriculaires.

12. Utilisation d'un composé de formule I selon une ou plusieurs des revendications 1 à 3 et/ou d'un sel physiologiquement acceptable d'un tel composé, pour la fabrication d'un médicament destiné à la thérapie ou à la prophylaxie de troubles du rythme cardiaque auxquels il est possible de remédier par prolongement du potentiel d'action.

13. Utilisation d'un composé de formule I selon une ou plusieurs des revendications 1 à 3 et/ou d'un sel physiologiquement acceptable d'un tel composé, pour la fabrication d'un médicament destiné à la thérapie ou à la prophylaxie de la fibrillation auriculaire ou du flutter auriculaire.

14. Utilisation d'un composé de formule I selon une ou plusieurs des revendications 1 à 3 et/ou d'un sel physiologiquement acceptable d'un tel composé, pour la fabrication d'un médicament destiné à la thérapie ou à la prophylaxie d'arythmies de réentrée ou à empêcher la mort subite par arrêt cardiaque, suite à une fibrillation ventriculaire.

15. Utilisation d'un composé de formule I selon une ou plusieurs des revendications 1 à 3 et/ou d'un sel physiologiquement acceptable d'un tel composé, pour la fabrication d'un médicament destiné à la thérapie de l'insuffisance cardiaque.
